# EUROPEAN PATENT SPECIFICATION

(11) **EP 4 157 406 B1**
(45) Date of publication and mention of the grant of the patent: **27.11.2024**
(21) Application number: 21734490.2
(22) Date of filing: 26.05.2021
(51) Int. Cl.: A61M 11/04, A24F 40/10, A24F 40/42, A24F 40/44, A61M 15/00, A61M 15/06, A24F 40/485, A24F 40/50

(54) **AEROSOL GENERATION DEVICE WITH A VALVE POSITION DETECTOR AND A DRYING CYCLE**
AEROSOLERZEUGUNGSVORRICHTUNG MIT EINEM VENTILSTELLUNGSDETEKTOR UND EINEM TROCKNUNGSZYKLUS
DISPOSITIF DE GÉNÉRATION D'AÉROSOL COMPRENANT UN DÉTECTEUR DE POSITION DE SOUPAPE ET UN CYCLE DE SÉCHAGE

(30) Priority: 28.05.2020 EP 20177240
(43) Date of publication of application: 05.04.2023
(73) Proprietor: JT International SA, 1202 Geneva (CH)
(72) Inventor: ZOMINY, Claude, 74350 Copponex (FR)
(74) Representative: Weihs, Bruno Konrad
(86) International application number: PCT/IB2021/054589
(87) International publication number: WO 2021/240394

(56) References cited:
- WO-A1-2013/083631
- WO-A1-2019/116004
- US-A1- 2017 233 114
- US-A1- 2017 280 773
- US-A1- 2019 364 957

## Description

### FIELD OF THE INVENTION

The present disclosure relates generally to an aerosol or vapor generation system and device, more particularly to an aerosol or vaporgeneration device with a removable capsule or other liquid-containing device for removable connection to the aerosol or vapor generating device, and to specific leaking avoidance aspects at the time of removing the removable capsule from the aerosol or vapor generation device.

### BACKGROUND

The use of aerosol generating systems, also known as e-cigarettes, e-cigs (EC), electronic nicotine delivery systems (ENDS), electronic non-nicotine delivery systems (ENNDS), electronic smoking devices (ESDs), personal vaporizers (PV), inhalation devices, vapes, which can be used as an alternative to conventional smoking articles such as lit-end cigarettes, cigars, and pipes, is becoming increasingly popular and widespread. The most commonly used e-cigarettes are usually battery powered and use a resistance heating element to heat and atomize a vaporizable material such as a liquid containing nicotine and/or flavorants (also known as e-cigarette liquid, e-cig liquids, e-liquid, juice, vapor juice, smoke juice, e-juice, e-fluid, vape oil), to produce an aerosol (often called vapor) which can be inhaled by a user.

In the conventional e-cigarettes described above, the liquid is put into contact with a resistance heating element after flowing through small channels, where it is heated and vaporized. The flowing is realized for example via a wick, a mesh or anothertype of porous element, which has a plurality of small channels that transport the liquid from a reservoir to the heating element. This heating element together with the porous element, a reservoir that contains the e-liquid, and a mouthpiece are usually arranged within a disposable capsule, cartridge or pod, that is discarded or refilled once the e-liquid has been consumed by the user, and usually removably connects to a main body that includes a rechargeable battery.

When the capsule, cartridge or pod needs to be removed from the aerosol generation device, some e-liquid may remain inside of it, especially in the wick, mesh or porous material. Remaining e-liquid may leak out from the mesh and flow anywhere inside the capsule and possibly out of the capsule into a user's pocket, a user's bag or directly onto the user.

Prior art publication U.S. Patent Publication No. 2017/0280773 A1, this reference discloses an aerosol-generating system that may comprise a releasably connectable capsule and vaporizing unit. The capsule may comprise a reservoir for containing an e-liquid, an opening in fluidic communication with the reservoir, and a valve configured to control a flow of the e-liquid from the reservoir through the opening. This publication however is silent about any means to prevent e-liquid leaks.

Consequently, the background art presents a number of deficiencies and problems and the present disclosure seeks to address these difficulties.

One aim of the invention is to address the issue of preventing e-liquid or vaporizable material from flowing inside the capsule and possibly out of the capsule onto the user.

### SUMMARY

The invention is defined by the appended claims.

Preferably, the aerosol generation device comprises a wicking element, a heating assembly configured to heat the wicking element, and a receiving interface element configured to receive a removable capsule comprising a valve. The aerosol generation device further comprises a valve detector configured to operate a drying cycle of the wicking element upon detection of a valve position by a valve detector.

According to another aspect of the present invention, the valve detector is further configured to detect if the valve is in the closed position at a time when the removable capsule is being removed from the aerosol generation device.

According to another aspect of the present disclosure, the valve detector is activated by a mechanical movement, preferably by a rotation of an annular element arranged on aerosol generation device.

According to yet another aspect of the present invention, the valve detector comprises at least one reflective zone, at least one light emitter and at least one light receiver.

According to another aspect of the disclosure, the heating assembly comprises a heater, a battery and a controller all in operative connection.

According to still another aspect of the present disclosure, the heating assembly is configured to trigger a drying cycle intended to evaporate the vaporizable material remaining in the wicking element when the valve is in the closed position.

According to another aspect of the present invention, the valve detector is configured to induce the heating assembly to trigger the drying cycle when the valve is in the closed position.

According to still another aspect of the present invention, the heating assembly is configured to heat the wicking element during the drying cycle.

According to another aspect of the present disclosure, during the drying cycle, the heating assembly is configured to operate at a temperature maintained between a temperature necessary to evaporate the vaporizable material and a temperature at which the wicking element would degrade.

According to yet another aspect of the present invention, the heating assembly further comprises a built-in delay configured to cool the wicking element before the end of the drying cycle.

According to still another aspect of the present disclosure, the aerosol generation device further comprises a visual signal triggered at the end of the drying cycle.

According to another aspect of the present invention, an aerosol generation system comprising the device as defined in the preceding description, and a removable capsule comprising a valve, the removable capsule is configured to contain a vaporizable material and having an aperture through which the vaporizable material can flow. The valve has a closed position in which the aperture is closed to retain the vaporizable material in the removable capsule and an open position in which the aperture is open to release the vaporizable material from the removable capsule toward the wicking element.

According to another aspect of the invention, the valve is configured to transition from the closed position to the open position when the removable capsule is inserted on the aerosol generation device.

According to another aspect of the invention, the valve is further configured to transition from the open position to the closed position when the removable capsule is removed from the aerosol generation device.

It is another aspect of the present invention to provide a method to perform a drying cycle for an aerosol generation device according to the present disclosure comprising steps of:
- closing the valve arranged on the removable capsule;
- activating the valve detector by a mechanical movement of the removable capsule to detect if the valve is in the closed position;
- heating the wicking element to evaporate the vaporizable material remaining in the wicking element.

According to yet another aspect of the present disclosure, the mechanical movement comprises the rotation of an annular element arranged on the removable capsule.

According to still another aspect of the present invention, the method further comprises a step of cooling the wicking element.

According to yet another aspect of the present invention, the method further comprises a step of triggering a visual signal indicating an end of the drying cycle.

According to still another aspect of the present disclosure, the method further comprises a step of removing the removable capsule.

The above and other objects, features and advantages of the present invention and the manner of realizing them will become more apparent, and the invention itself will best be understood from a study of the following detailed description of preferred embodiments of the invention, with reference to the attached drawings showing some preferred embodiments of the invention.

### BRIEF DESCRIPTION OF THE DRAWINGS

The accompanying drawings, which are incorporated herein and constitute part of this specification, illustrate the presently preferred embodiments of the invention, and together with the general description given above and the detailed description given below, serve to explain features of the invention.
Figure 1 shows a schematic view of a base of an aerosol generation device and a removable capsule comprising a valve in a closed position according to an example embodiment of the invention;
Figure 2 shows the removable capsule of the aerosol generation device from figure wherein the removable capsule is inserted on the aerosol generation device and the valve is in an open position;
Figure 3 shows a view of a removable capsule comprising a valve in a closed position and a valve detector arranged on the removable capsule according to a further example embodiment of the invention;
Figure 4 depicts the removable capsule of figure 3 in which the valve is in an open position;
Figure 5 depicts a valve in the form of a disc according to a further example embodiment of the invention;
Figure 6 is a cross-sectional schematic view of a valve in the closed position according to the further example embodiment of the invention;
Figure 7 is a cross-sectional schematic view of a valve in the open position according to the further example embodiment of the invention;
Figure 8 is a flow-chart of a drying cycle method according to a further example embodiment of the invention;
Figure 9 is a flow-chart of a drying cycle method according to yet another example embodiment of the invention;
Figure 10 is a flow-chart of a drying cycle method according to another example embodiment of the invention; and
Figure 11 is a flow-chart of a drying cycle method according to another example embodiment of the invention.

Identical reference numerals are used, where possible, to designate identical elements that are common throughout the figures. Also, the images are simplified for illustration purposes and may not be depicted to scale.

### DETAILED DESCRIPTION OF SEVERAL EMBODIMENTS

We will from now on use the term "wicking element" to designate also any alternative also encompassing a wick, a mesh or other porous material alternative, and the term "capsule" to designate also any alternative encompassing a cartridge or a pod.

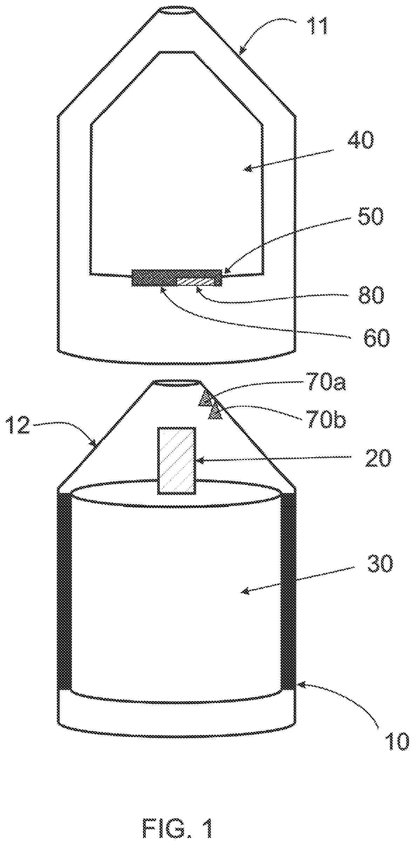

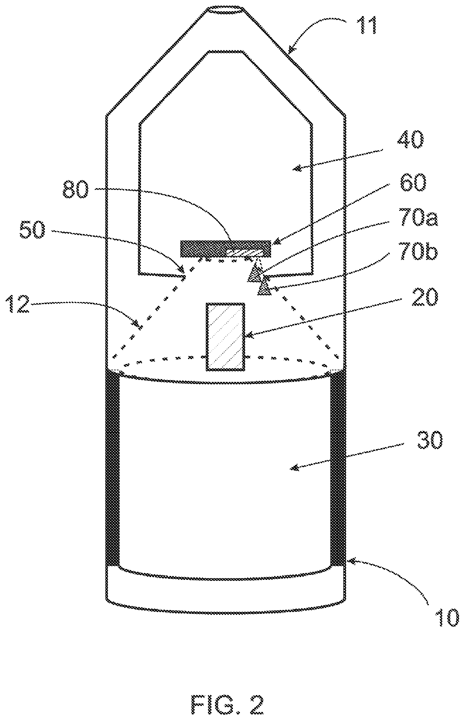

Figures 1 and 2 show an aerosol generation device 10 and a removable capsule 11.

The aerosol generation device 10 may have a cylindrical or parallelepipedal geometry and be made from plastic, metallic or any suitable material and may be fabricated as a single piece or multiple pieces using one or several of the mentioned materials.

The aerosol generation device 10 comprises a receiving interface element 12 configured to receive the removable capsule 11 with a valve 60. The receiving interface element 12 is represented in figure 1 in a schematic manner only.

The aerosol generation device 10 may comprise a power source such as a rechargeable battery in operative connection with a controller, for example one from the list comprising a microcontroller, a microprocessor, a data processor, an electronic circuit that allows to control and monitor the power source. These elements are not illustrated in figure 1.

The aerosol generation device 10 may further comprise a first electrical connector in operative connection with the controller and the power source, hence enabling the electrical connection of the aerosol generation device 10 to the removable capsule 11 (not shown in figure 1).

The aerosol generation device 10 may also comprise a first fixation system (not shown in figure 1) configured to hold the removable capsule 11 on the aerosol generation device 10 and to release the removable capsule 11 from the aerosol generation device 10 when the removable capsule 11 needs to be removed.

The aerosol generation device 10 also comprises a valve detector 70a, 70b configured to sense a position of the valve 60 when the removable capsule is received on the receiving interface 12.

The removable capsule 11 may comprise a second electrical connector (not shown in figure 1) which is configured to connect to the first electrical connector from the aerosol generation device 10 when the removable capsule 11 is fixed on the aerosol generation device 10.

The removable capsule 11 may also comprise a second fixation system (not shown in figure 1) configured to fix the removable capsule 11 to the aerosol generation device 10 through the first fixation system arranged on the aerosol generation device 10.

As schematically depicted in figures 1 and 2, the aerosol generation device 10 comprises a wicking element 20 and a heating assembly 30 in operative connection with the wicking element 20 and configured to heat the wicking element 20.

The heating assembly 30 may comprise a heater, a battery and a controller all in operative connection.

The removable capsule 11 comprises an internal cavity or reservoir 40 configured to contain a vaporizable material which can be heated to produce an aerosol for inhalation by a user.

The term vaporizable material is used to designate any material that is vaporizable at a temperature up to 400°C, preferably up to 350°C, for example aerosol generating liquid, gel, wax and the like.

In another embodiment, not illustrated in the figures, the wicking element is arranged on the removable capsule 11 and is in operative connection with the heating assembly 30 arranged on the aerosol generation device 10 when the removable capsule 11 is connected to the aerosol generation device 10. In this embodiment, the wicking element 20 on the aerosol generation device 10 may likely be removed since redundant with the wicking element on the removable capsule.

In another embodiment, not illustrated in the figures, the removable capsule 11 may also comprises the heating assembly in operative connection with the wicking element and configured to heat the wicking element. The heater may be in operative connection with the second electrical connector arranged on the removable capsule 11 and is therefore electrically connected to the controller and the power source comprised in the aerosol generation device 10 when the removable capsule 11 is connected to the aerosol generation device 10.

The removable capsule 11 comprises an aperture 50 arranged in connection with the reservoir 40 and through which the vaporizable material may flow, for example toward the wicking element 20.

The aperture 50 may be arranged at any suitable location in the removable capsule 11 in connection with the reservoir 40 in order to allow the vaporizable material to flow toward the wicking element 20.

The aperture 50 may be circular or rectangular or of any different suitable geometry.

The aperture 50 may have an opening surface suitable to allow a correct flow of the vaporizable material toward the wicking element 20 when the capsule 11 is inserted on the aerosol generation device 10.

In another embodiment the removable capsule 11 may comprise more than one aperture 50, each of the apertures being in connection with the reservoir 40 and being enabled to let the vaporizable material flow through it toward the wicking element 20.

The removable capsule 11 further comprises a closing element arranged on the aperture 50. The closing element may be for example the valve 60.

The valve 60 is arranged on the aperture 50. The valve 60 has a closed position in which the valve 60 closes the aperture 50 to retain the vaporizable material in in the reservoir 40 and an open position in which the aperture 50 is open to release the vaporizable material from the reservoir 40 toward the wicking element 20.

The valve 60 has a geometry and a size allowing to sealingly close the aperture 50 when the valve 60 is in the closed position in order to retain the vaporizable material inside the reservoir 40.

The removable capsule 11 may further comprise sealing means arranged at the periphery of the aperture 50 configured to seal the closure of the aperture 50 by the valve 60, when the valve 60 is in the closed position. The sealing means may comprise at least one gasket 110.

Alternatively, the sealing means may also be arranged on the valve 60.

The valve 60 is further configured to transition from the closed position to the open position when the removable capsule 11 is inserted on the aerosol generation device 10.

The valve 60 is further configured to transition from the open position to the closed position when the removable capsule 11 is removed on the aerosol generation device 10.

In another embodiment, the removable capsule 11 may comprise more than one valve 60 if the removable capsule 11 comprises more than one aperture 50. One valve 60 being arranged on one aperture 50 (not illustrated in the figures).

In another embodiment, the removable capsule 11 may comprise one valve 60 configured to close several apertures 50.

As depicted in figures 1 and 2, the aerosol generation device comprises the valve detector 70a, 70b configured to detect the position of the valve 60.

The valve detector 70a, 70b is further configured to detect if the valve 60 is in the closed position or in the open position when the removable capsule 11 is received on the receiving interface element 12.

The valve detector 70a, 70b is further configured to detect if the valve 60 is in the closed position at the time when the removable capsule 11 is being removed from the aerosol generation device 10.

The valve detector 70a, 70b may be activated by a mechanical movement of the removable capsule 11.

The valve detector 70a, 70b may be activated by a user when the user wants to remove the removable capsule 11.

The valve detector 70a, 70b further comprises or cooperate with at least one reflective zone 80 arranged on the valve 60. The valve detector 70a, 70b further comprises at least one light emitter 70a and at least one light receiver 70b.

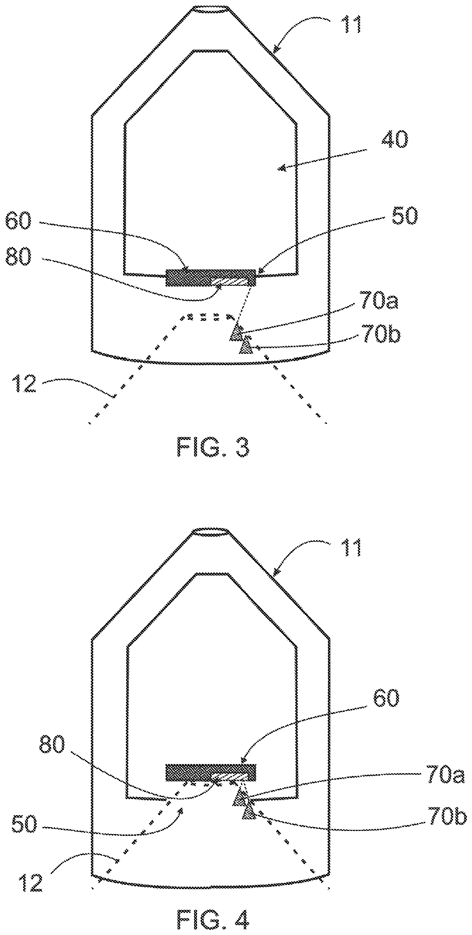

As depicted in figure 3, representing the receiving interface element 12 relative to the removable capsule, when the valve 60 is in the closed position, the at least one reflective zone 80 is in a position in which a light signal emitted from light emitter 70a may not reach the at least one reflective zone 80, and no light may be reflected by the latterto reach the at least one light receiver 70b, indicating that the valve 60 is in the closed position.

As depicted on figure 4, representing the receiving interface element relative to the removable capsule, when the valve 60 is in the open position, the at least one reflective zone 80 is in a position such that the at least one light emitter 70a may emit light that is then reflected from the at least one reflective zone 80 towards the at least one light receiver 70b. In the open position, when the valve detector 70a, 70b is activated, the at least one light emitter 70a emits the light signal. The light signal is reflected by the at least one reflective zone 80 arranged on the valve 60 in direction of the at least one light receiver 70b. The at least one light receiver 70b can therefore capture this reflected light indicating that the valve 60 is in the open position.

In another embodiment not illustrated, the aerosol generation device 10 further comprises an annular element configured to activate the valve detector 70a, 70b. The detector may be activated by a rotation of the annular element arranged on the aerosol generation device 10. The rotation of the annular element may be performed by a user at the time when the removable capsule 11 needs to be removed from the aerosol generation device 10. The rotation of the annular element may further enable an actuation of the valve from an open position to a closed position or the otherway around.

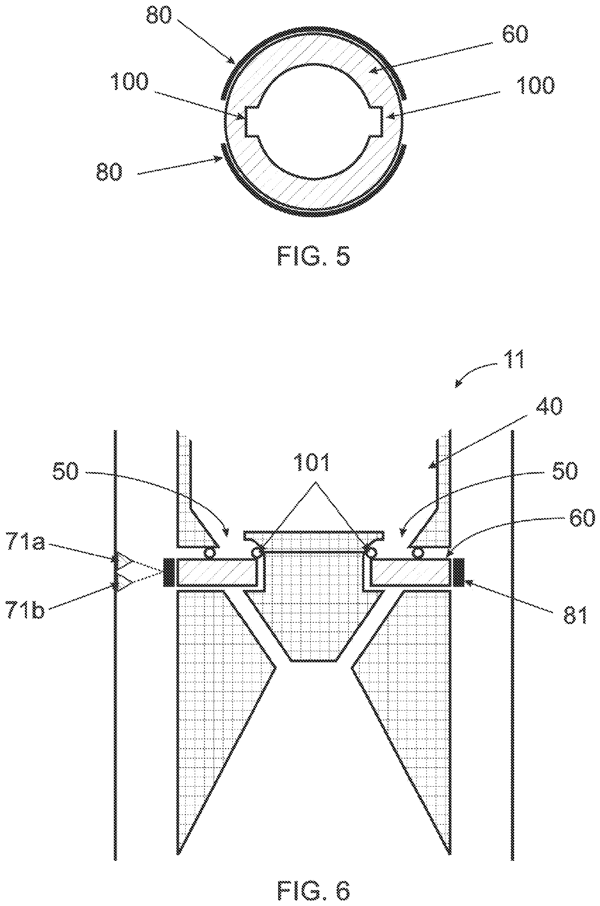

In another embodiment according to FIGs 5 to 7, the removable capsule 11 comprises two apertures 50 and a closing element or a valve in the form of a ring 60.

The ring 60 is configured to rotate around a central axis of the removable capsule 11 from an open position to a closed position or the other way around.

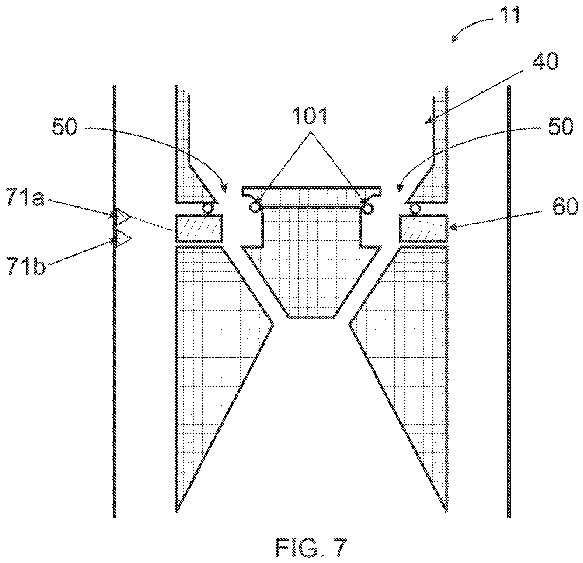

As depicted in figure 5, the ring 60 may comprise two recesses or openings 100 arranged preferentially on the inner side of the ring 60 and configured to be aligned with two apertures 50 arranged on the removable capsule 11 when the ring 60 is in the open position (figure 7) hence enabling the vaporizable material to flow from the reservoir 40 toward the wicking element via the two apertures 50 (the wicking element is not represented on figure 5).

The ring 60 further comprises two reflective zones 81 arranged on the ring 60. The reflective zones 80 are arranged on the external side of the ring 60 excepting the areas of the external side of the ring 60 which are facing the two recesses 100 arranged on the inner side of the ring 60.

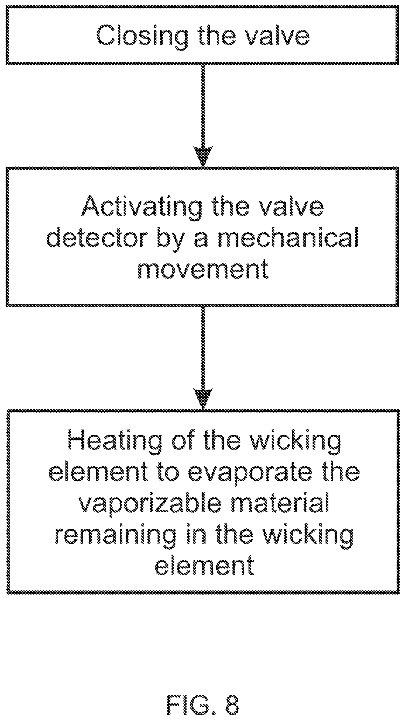

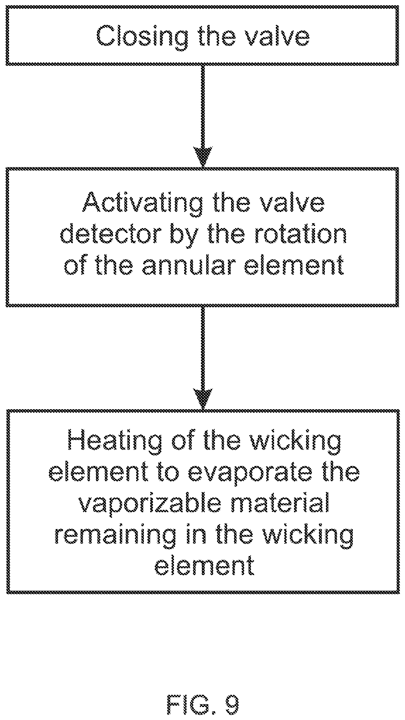

The ring 60 is configured to rotate around the central axis of the removable capsule 11 from a closed position (figure 8) to an open position (figure 9).

The ring 60 may be rotated by a user moving it from the closed to the open position or the other way around.

As depicted in figure 6, when the ring 60 is in the closed position, the ring 60 closes the two apertures 50 arranged on the removable capsule. The vaporizable material is hence retained in the reservoir of the removable capsule.

A valve detector 71a, 71b is configured to determine if the valve 60 is in the closed position.

When the ring 60 is in the closed position, at least one of the reflective zones 81 is facing the at least one light emitter 71a and the at least one light receiver 71b, meaning that a light signal emitted from light emitter 71a may reach the at least one of the reflective zones 81, and light reflected by the latter may reach the at least one light receiver 71b. In the closed position, when the valve detector 71a, 71b is activated, the at least one light emitter 71a emits the light signal. The light signal is reflected by the at least one reflective zone 81 arranged on the ring 60 in direction of the at least one light receiver 71b. The at least one light receiver 71b can therefore capture this reflected light indicating that the ring 60 is in the closed position.

As shown on figure 7, when the ring is in the open position, the recesses are aligned with the apertures 50 of the removable capsule. The apertures are hence open, and the vaporizable material can flow from the reservoir toward the wicking element via the two apertures.

When the ring 60 is in the open position, the at least one of the reflective zones 81 is not facing the at least one light emitter 71a and the at least one light receiver 71b. In the open position, when the at least one light emitter 71a emits the light signal, and the light signal is not reflected by the at least one reflective zone 81 arranged on the ring 60 as the at least one of the reflective zones 81 is not facing the at least one light emitter 71a. The at least one light receiver 71b cannot therefore capture this reflected light indicating that the ring 60 is in the closed position.

When the valve detector 71a, 71b detects or determines that the valve 60 is in the closed position, the valve detector 71a, 71b is configured to induce the heating assembly 30 to trigger a drying cycle when the valve 60 is in the closed position.

The drying of the vaporizable material remaining in the wicking element 20 by the heating assembly 30 should be performed when the valve or the ring 60 is in the closed position to avoid vaporizable material releasing or leaking from the capsule 11 when the drying cycle is operating.

The heating assembly may be configured to operate a drying cycle of the wicking element 20 upon detection of a valve position by a valve detector (70a, 70b, 71a, 71b).

The drying cycle may be triggered by the heating assembly 30 to dry the remaining vaporizable material in the wicking element 20 when the valve 60 is in the closed position.

The drying cycle is intended to evaporate the remaining vaporizable material in the wicking element 20, without burning ordamaging the wicking element 20 by heating the wicking element 20 at an appropriate low power for a certain period of time.

The drying cycle is also indented to evaporate the remaining vaporizable material in the wicking element 20, without overheating and burning the vaporizable material as this may generate undesirable substances in the wicking element.

During the drying cycle, the heating assembly 30 is configured to heat the wicking element 20 to the end of evaporating any vaporizable material remaining in the wicking element 20.

The heating assembly 30 is further configured to heat the wicking element 20 at a temperature maintained between a temperature necessary to evaporate the vaporizable material remaining in the wicking element 20 and a temperature at which the wicking element 20 would degrade.

The heating assembly 30 is further configured to heat the wicking element 20 during the drying cycle at a boiling temperature of the vaporizable material.

The aerosol generation device may also comprise a wicking element temperature sensor (not represented) configured to measure the wicking element temperature to avoid reaching a too high temperature that would damage the wicking element.

The aerosol generation device may also comprise a wicking element resistance sensor (not represented) configured to measure the wicking element temperature to avoid reaching a too high temperature that would damage the wicking element sensor. Such resistance sensor may in particular be implemented with a wicking element formed of an at least partially resistant metallic mesh.

To avoid damaging the wicking element 20, the heating assembly 30 may be configured, for example, to heat the wicking element 20 for 0.5 to 5 seconds.

The heating assembly 30 may further comprise a built-in-delay configured to cool the wicking element 20 before the end of the drying cycle.

The built-in-delay is configured to cool the wicking element 20, for example, between 5 to 10 seconds enabling a complete cooling of the wicking element 20. The built-in-delay is configured to cool the wicking element until the wicking element temperature is approximately 50°C. The temperature of the wicking element may be monitored by the wicking element temperature sensor or the wicking element resistance sensor.

In another embodiment, the wicking element 20 may be cooled by passive or active cooling.

The aerosol generation device 10 may further comprise a visual signal (not represented) triggered at the end of the drying cycle, indicating to the user that the drying cycle is finished.

The visual signal may comprise a LED or a luminous device emitting a light signal at the end of the drying cycle.

As depicted in figure 8, it is another object of the invention to provide a method to perform a drying cycle configured to dry the wicking element 20 without damaging it, comprising steps of:
- -closing the valve 60 arranged on the removable capsule 11;

- activating the valve detector 70a, 70b by a mechanical movement to detect if the valve 60 is in the closed position;
- heating the wicking element 20 to evaporate the vaporizable material remaining in the wicking element 20.

The activation of the valve detector 7 in the method to perform a drying cycle may further comprise a rotation of an annular element arranged on the aerosol generation device, as depicted in figure 9.

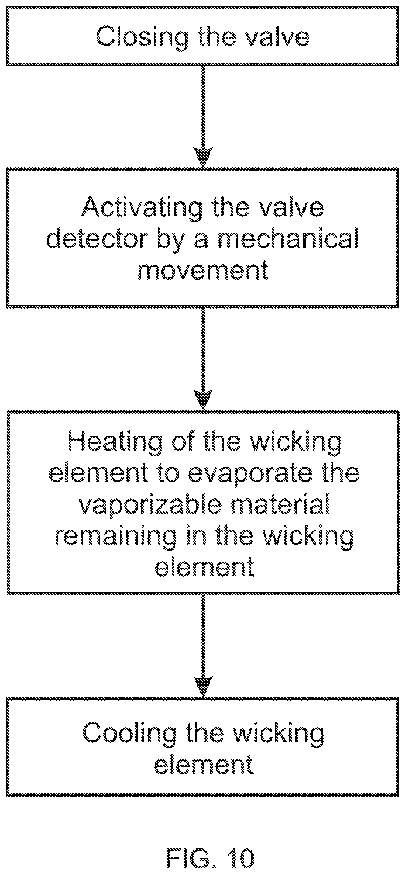

The method to perform a drying cycle may further comprise the step of cooling the wicking element 20, for example via a built-in-delay configured to cool the wicking element 20 as shown in figure 10.

The method to perform a drying cycle may further comprise the step of triggering a visual signal indicating an end of the drying cycle.

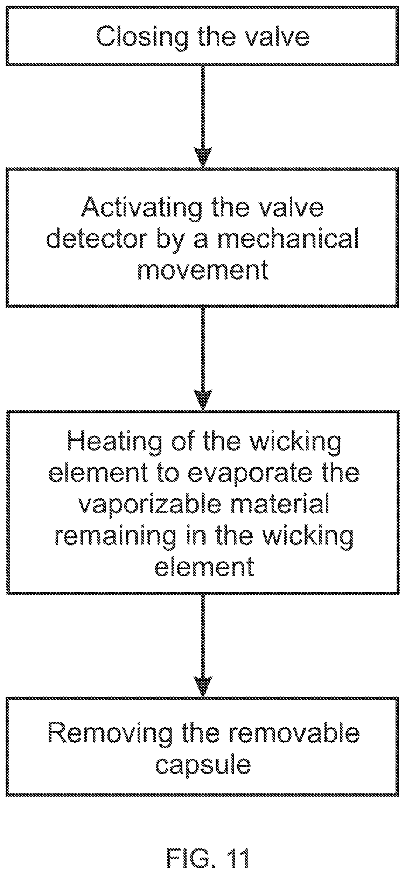

The method to perform a drying cycle may further comprise the step of removing the removable capsule 11 at the end of the drying cycle as depicted in figure 11.

The details and embodiments described above for an aerosol generation device 10 are applicable to an electronic cigarette comprising a removable capsule 11.

While the invention has been disclosed with reference to certain preferred embodiments, numerous modifications, alterations, and changes to the described embodiments, and equivalents thereof, are possible without departing from the scope of the claims.

Accordingly, it is that the invention not be limited to the described embodiments and be given the broadest reasonable interpretation in accordance with the language of the appended claims.

## Claims

1. An aerosol generation device (10) comprising:
- a wicking element (20),
- a heating assembly (30) configured to heat the wicking element (20),
- a receiving interface element (12) configured to receive a removable capsule (11) configured to contain a vaporizable material and having an aperture (50) through which the vaporizable material can flow, the removable capsule (11) comprising a valve (60) arranged on the aperture (50), the valve (60) having a closed position in which the valve (60) closes the aperture (50) and an open position in which the aperture (50) is open to release the vaporizable material toward the wicking element (20),
- a valve detector (70a, 70b) configured to detect if the valve (60) is in the closed position or in the open position when the removable capsule (11) is received on the receiving interface element (12), **characterized in that** the heating assembly (30) is configured to operate a drying cycle of the wicking element (20) upon detection of a valve (60) position by the valve detector (70a, 70b).

2. The aerosol generation device (10) as defined in claim 1, wherein the valve detector (70a, 70b) is further configured to detect if the valve (60) is in the closed position at a time when the removable capsule (11) is being removed from the aerosol generation device.

3. The aerosol generation device (10) as defined in any one of the preceding claims, wherein the valve detector (70a, 70b) is activated by a mechanical movement, preferably by a rotation of an annular element arranged on the aerosol generation device (10).

4. The aerosol generation device (10) as defined in any one of the preceding claims, wherein the valve detector (70a, 70b) comprises at least one reflective zone (80), at least one light emitter (70a) and at least one light receiver (70b).

5. The aerosol generation device (10) as defined in any one of the preceding claims, wherein the heating assembly (30) comprises a heater, a battery and a controller all in operative connection.

6. The aerosol generation device (10) as defined in any one of the preceding claims, wherein the heating assembly (30) is configured to trigger a drying cycle intended to evaporate the vaporizable material remaining in the wicking element (20) when the valve (60) is in the closed position.

7. The aerosol generation device (10) as defined in any one of the preceding claims, wherein the valve detector 70 is configured to induce the heating assembly (30) to trigger the drying cycle when the valve (60) is in the closed position.

8. The aerosol generation device (10) as defined in any one of the preceding claims, wherein the heating assembly (30) is configured to heat the wicking element (20) during the drying cycle.

9. The aerosol generation device (10) as defined in any one of the preceding claims, wherein during the drying cycle, the heating assembly (30) is configured to operate at a temperature maintained between a temperature necessary to evaporate the vaporizable material and a temperature at which the wicking element (20) would degrade.

10. The aerosol generation device (10) as defined in any one of the preceding claims, wherein the heating assembly (30) further comprises a built-in delay configured to cool the wicking element (20) before the end of the drying cycle.

11. The aerosol generation device (10) as defined in any one of the preceding claims, wherein the aerosol generation device (10) further comprises a visual signal triggered at the end of the drying cycle.

12. An aerosol generation system comprising the device as defined in the preceding claims, and a removable capsule (11) comprising a valve (60), the removable capsule (11) is configured to contain a vaporizable material and having an aperture (50) through which the vaporizable material can flow, the valve (60) having a closed position in which the aperture (50) is closed to retain the vaporizable material in the removable capsule (11) and an open position in which the aperture (50) is open to release the vaporizable material from the removable capsule (11) toward the wicking element (20).

13. The aerosol generation system according to the preceding claim,
wherein the valve (60) is configured to transition from the closed position to the open position when the removable capsule (11) is inserted on the aerosol generation device.

14. The aerosol generation system according to claim 12 or 13, wherein the valve (60) is further configured to transition from the open position to the closed position when the removable capsule (11) is removed from the aerosol generation device.

15. A method to perform a drying cycle for an aerosol generation device (10) according to claim 1 comprising steps of:
- closing the valve (60) arranged on the removable capsule (11),
- activating the valve detector (70a, 70b) by a mechanical movement of the removable capsule (11) to detect if the valve (60) is in the closed position,
- heating the wicking element (20) to evaporate the vaporizable material remaining in the wicking element (20).

## Patentansprüche

1. Aerosolerzeugungsvorrichtung (10), umfassend:
- ein Dochtelement (20),
- eine Heizanordnung (30), die dazu ausgelegt ist, das Dochtelement (20) zu erhitzen,
- ein Aufnahmeschnittstellenelement (12), das dazu ausgelegt ist, eine herausnehmbare Kapsel (11) aufzunehmen, die dazu ausgelegt ist, ein verdampfbares Material zu enthalten, und eine Öffnung (50) aufweist, durch die das verdampfbare Material fließen kann, wobei die herausnehmbare Kapsel (11) ein Ventil (60) umfasst, das an der Öffnung (50) angeordnet ist, wobei das Ventil (60) eine Schließposition, in der das Ventil (60) die Öffnung (50) verschließt, und eine Öffnungsposition aufweist, in der die Öffnung (50) geöffnet ist, um das verdampfbare Material in Richtung des Dochtelements (20) freizugeben,
einen Ventildetektor (70a, 70b), der dazu ausgelegt ist zu erkennen, ob sich das Ventil (60) in der Schließposition oder Öffnungsposition befindet, wenn die herausnehmbare Kapsel (11) auf dem Aufnahmeschnittstellenelement (12) aufgenommen wird,
**dadurch gekennzeichnet, dass** die Heizanordnung (30) dazu ausgelegt ist, einen Trocknungszyklus des Dochtelements (20) durchzuführen, wenn eine Position des Ventils (60) durch den Ventildetektor (70a, 70b) erkannt wird.

2. Aerosolerzeugungsvorrichtung (10) nach Anspruch 1, wobei der Ventildetektor (70a, 70b) ferner dazu ausgelegt ist zu erkennen, ob sich das Ventil (60) in der Schließposition befindet, wenn die herausnehmbare Kapsel (11) aus der Aerosolerzeugungsvorrichtung entfernt wird.

3. Aerosolerzeugungsvorrichtung (10) nach einem der vorhergehenden Ansprüche, wobei der Ventildetektor (70a, 70b) durch eine mechanische Bewegung aktiviert wird, vorzugsweise durch eine Drehung eines ringförmigen Elements, das an der Aerosolerzeugungsvorrichtung (10) angeordnet ist.

4. Aerosolerzeugungsvorrichtung (10) nach einem der vorhergehenden Ansprüche, wobei der Ventildetektor (70a, 70b) mindestens eine reflektierende Zone (80), mindestens einen Lichtemitter (70a) und mindestens einen Lichtempfänger (70b) umfasst.

5. Aerosolerzeugungsvorrichtung (10) nach einem der vorhergehenden Ansprüche, wobei die Heizanordnung (30) ein Heizelement, eine Batterie und eine Steuereinheit umfasst, die alle in Wirkverbindung stehen.

6. Aerosolerzeugungsvorrichtung (10) nach einem der vorhergehenden Ansprüche, wobei die Heizanordnung (30) dazu ausgelegt ist, einen Trocknungszyklus auszulösen, der dazu bestimmt ist, das im Dochtelement (20) verbleibende verdampfbare Material zu verdampfen, wenn sich das Ventil (60) in der Schließposition befindet.

7. Aerosolerzeugungsvorrichtung (10) nach einem der vorhergehenden Ansprüche, wobei der Ventildetektor (70) dazu ausgelegt ist, die Heizanordnung (30) zu veranlassen, den Trocknungszyklus auszulösen, wenn sich das Ventil (60) in der Schließposition befindet.

8. Aerosolerzeugungsvorrichtung (10) nach einem der vorhergehenden Ansprüche, wobei die Heizanordnung (30) dazu ausgelegt ist, das Dochtelement (20) während des Trocknungszyklus zu erhitzen.

9. Aerosolerzeugungsvorrichtung (10) nach einem der vorhergehenden Ansprüche, wobei die Heizanordnung (30) während des Trocknungszyklus dazu ausgelegt ist, bei einer Temperatur zu arbeiten, die zwischen einer Temperatur, die zum Verdampfen des verdampfbaren Materials erforderlich ist, und einer Temperatur, bei der sich das Dochtelement (20) zersetzen würde, gehalten wird.

10. Aerosolerzeugungsvorrichtung (10) nach einem der vorhergehenden Ansprüche, wobei die Heizanordnung (30) ferner eine eingebaute Verzögerung umfasst, die dazu ausgelegt ist, das Dochtelement (20) vor dem Ende des Trocknungszyklus abzukühlen.

11. Aerosolerzeugungsvorrichtung (10) nach einem der vorhergehenden Ansprüche, wobei die Aerosolerzeugungsvorrichtung (10) ferner ein visuelles Signal umfasst, das am Ende des Trocknungszyklus ausgelöst wird.

12. Aerosolerzeugungssystem, umfassend die Vorrichtung nach einem der vorhergehenden Ansprüche und eine herausnehmbare Kapsel (11) mit einem Ventil (60), wobei die herausnehmbare Kapsel (11) dazu ausgelegt ist, ein verdampfbares Material zu enthalten und eine Öffnung (50) aufweist, durch die das verdampfbare Material fließen kann, wobei das Ventil (60) eine Schließposition, in der die Öffnung (50) geschlossen ist, um das verdampfbare Material in der herausnehmbaren Kapsel (11) zurückzuhalten, und eine Öffnungsposition aufweist, in der die Öffnung (50) geöffnet ist, um das verdampfbare Material aus der herausnehmbaren Kapsel (11) in Richtung des Dochtelements (20) freizugeben.

13. Aerosolerzeugungssystem nach dem vorhergehenden Anspruch,
wobei das Ventil (60) dazu ausgelegt ist, aus der Schließposition in die Öffnungsposition zu wechseln, wenn die herausnehmbare Kapsel (11) in die Aerosolerzeugungsvorrichtung eingesetzt wird.

14. Aerosolerzeugungssystem nach Anspruch 12 oder 13, wobei das Ventil (60) ferner dazu ausgelegt ist, aus der Öffnungsposition in die Schließposition zu wechseln, wenn die herausnehmbare Kapsel (11) aus der Aerosolerzeugungsvorrichtung entfernt wird.

15. Verfahren zur Durchführung eines Trocknungszyklus für eine Aerosolerzeugungsvorrichtung (10) nach Anspruch 1, umfassend die folgenden Schritte:
- Schließen des Ventils (60), das an der herausnehmbaren Kapsel (11) angeordnet ist,
- Aktivieren des Ventildetektors (70a, 70b) durch eine mechanische Bewegung der herausnehmbaren Kapsel (11), um zu erkennen, ob sich das Ventil (60) in der Schließposition befindet,
Erhitzen des Dochtelements (20), um das im Dochtelement (20) verbleibende verdampfbare Material zu verdampfen.

## Revendications

1. Dispositif de génération d'aérosol (10) comprenant :
- un élément mèche (20),
- un ensemble chauffant (30) configuré pour chauffer l'élément mèche (20),
- un élément d'interface de réception (12) configuré pour recevoir une capsule pouvant être retirée (11) configurée pour contenir un matériau vaporisable et ayant un orifice (50) à travers lequel le matériau vaporisable peut s'écouler, la capsule pouvant être retirée (11) comprenant une soupape (60) agencée sur l'orifice (50), la soupape (60) ayant une position fermée dans laquelle la soupape (60) ferme l'orifice (50) et une position ouverte dans laquelle l'orifice (50) est ouvert pour libérer le matériau vaporisable vers l'élément mèche (20),
- un détecteur de soupape (70a, 70b) configuré pour détecter si la soupape (60) est dans la position fermée ou dans la position ouverte lorsque la capsule pouvant être retirée (11) est reçue sur l'élément d'interface de réception (12),
**caractérisé en ce que** l'ensemble chauffant (30) est configuré pour faire fonctionner un cycle de séchage de l'élément mèche (20) lors de la détection d'une position de soupape (60) par le détecteur de soupape (70a, 70b).

2. Dispositif de génération d'aérosol (10) tel que défini dans la revendication 1, dans lequel le détecteur de soupape (70a, 70b) est en outre configuré pour détecter si la soupape (60) est dans la position fermée à un moment où la capsule pouvant être retirée (11) est en train d'être retirée du dispositif de génération d'aérosol.

3. Dispositif de génération d'aérosol (10) tel que défini dans l'une quelconque des revendications précédentes, dans lequel le détecteur de soupape (70a, 70b) est activé par un déplacement mécanique, préférablement par une rotation d'un élément annulaire agencé sur le dispositif de génération d'aérosol (10).

4. Dispositif de génération d'aérosol (10) tel que défini dans l'une quelconque des revendications précédentes, dans lequel le détecteur de soupape (70a, 70b) comprend au moins une zone réfléchissante (80), au moins un émetteur de lumière (70a) et au moins un récepteur de lumière (70b).

5. Dispositif de génération d'aérosol (10) tel que défini dans l'une quelconque des revendications précédentes, dans lequel l'ensemble chauffant (30) comprend un chauffage, une batterie et un dispositif de commande tous en connexion fonctionnelle.

6. Dispositif de génération d'aérosol (10) tel que défini dans l'une quelconque des revendications précédentes, dans lequel l'ensemble chauffant (30) est configuré pour déclencher un cycle de séchage destiné à évaporer le matériau vaporisable restant dans l'élément mèche (20) lorsque la soupape (60) est dans la position fermée.

7. Dispositif de génération d'aérosol (10) tel que défini dans l'une quelconque des revendications précédentes, dans lequel le détecteur de soupape 70 est configuré pour induire l'ensemble chauffant (30) à déclencher le cycle de séchage lorsque la soupape (60) est dans la position fermée.

8. Dispositif de génération d'aérosol (10) tel que défini dans l'une quelconque des revendications précédentes, dans lequel l'ensemble chauffant (30) est configuré pour chauffer l'élément mèche (20) pendant le cycle de séchage.

9. Dispositif de génération d'aérosol (10) tel que défini dans l'une quelconque des revendications précédentes, dans lequel pendant le cycle de séchage, l'ensemble chauffant (30) est configuré pour fonctionner à une température maintenue entre une température nécessaire pour évaporer le matériau vaporisable et une température à laquelle l'élément mèche (20) se dégraderait.

10. Dispositif de génération d'aérosol (10) tel que défini dans l'une quelconque des revendications précédentes, dans lequel l'ensemble chauffant (30) comprend en outre un délai intégré configuré pour refroidir l'élément mèche (20) avant la fin du cycle de séchage.

11. Dispositif de génération d'aérosol (10) tel que défini dans l'une quelconque des revendications précédentes, dans lequel le dispositif de génération d'aérosol (10) comprend en outre un signal visuel déclenché à la fin du cycle de séchage.

12. Système de génération d'aérosol comprenant le dispositif tel que défini dans les revendications précédentes, et une capsule pouvant être retirée (11) comprenant une soupape (60), la capsule pouvant être retirée (11) étant configurée pour contenir un matériau vaporisable et ayant un orifice (50) à travers lequel le matériau vaporisable peut s'écouler, la soupape (60) ayant une position fermée dans laquelle l'orifice (50) est fermé pour retenir le matériau vaporisable dans la capsule pouvant être retirée (11) et une position ouverte dans laquelle l'orifice (50) est ouvert pour libérer le matériau vaporisable de la capsule pouvant être retirée (11) vers l'élément mèche (20).

13. Système de génération d'aérosol selon la revendication précédente,
dans lequel la soupape (60) est configurée pour passer de la position fermée à la position ouverte lorsque la capsule pouvant être retirée (11) est insérée sur le dispositif de génération d'aérosol.

14. Système de génération d'aérosol selon la revendication 12 ou la revendication 13, dans lequel la soupape (60) est en outre configurée pour passer de la position ouverte à la position fermée lorsque la capsule pouvant être retirée (11) est retirée du dispositif de génération d'aérosol.

15. Procédé pour réaliser un cycle de séchage pour un dispositif de génération d'aérosol (10) selon la revendication 1 comprenant des étapes consistant à :
- fermer la soupape (60) agencée sur la capsule pouvant être retirée (11),
- activer le détecteur de soupape (70a, 70b) par un déplacement mécanique de la capsule pouvant être retirée (11) pour détecter si la soupape (60) est dans la position fermée,
- chauffer l'élément mèche (20) pour évaporer le matériau vaporisable restant dans l'élément mèche (20).
